(19) Europäisches Patentamt
European Patent Office
Office européen des brevets

(11) Publication number: **0 220 831 B1**

(12) **EUROPEAN PATENT SPECIFICATION**

(45) Date of publication of patent specification: **20.04.94**

(51) Int. Cl.[5]: **C08F 299/00**, C08F 12/34, C08G 65/00, C08G 75/04

(21) Application number: **86307335.9**

(22) Date of filing: **24.09.86**

(54) **Styryloxy resins and polymerization process.**

(30) Priority: **24.09.85 US 779737**

(43) Date of publication of application:
**06.05.87 Bulletin 87/19**

(45) Publication of the grant of the patent:
**20.04.94 Bulletin 94/16**

(84) Designated Contracting States:
**DE FR GB**

(56) References cited:
**EP-A- 0 008 795**
**EP-A- 0 165 769**
**US-A- 2 488 501**
**US-A- 3 663 625**
**US-A- 4 486 582**

**CHEMICAL ABSTRACTS, vol. 63, no. 8, 11th October 1965, column 9968 c-d,Columbus, Ohio, US; & JP-A-8552('65)(KYORIN PHARMACEUTICAL LTD) 04-05-1965**

(73) Proprietor: **LOCTITE (IRELAND) Ltd.**
**Whitestown Industrial Estate**
**Tallaght, Co. Dublin 24(IE)**

(72) Inventor: **Woods, John**
**138 Stillorgan Wood,**
**Upper Kilmacud Road**
**Stillorgan, Cy Dublin(IE)**
Inventor: **Harris, Stephen J.**
**10 Broadford Crescent**
**Ballinteer, Dublin(IE)**
Inventor: **Rooney, John**
**76, The Paddocks**
**Naas, Cy Kildare(IE)**

(74) Representative: **Marchant, James Ian**
**Elkington and Fife**
**Prospect House**
**8 Pembroke Road**
**Sevenoaks, Kent TN13 1XR (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

It is the object of the present invention to provide a new class of polymerizable monomers which are poly-functional, so as to be cured to crossed-linked high molecular weight polymer networks, and which are readily cationically polymerizable.

In USSR Patents 443874 and 478026 where are described ion exchange polymers prepared by free radical copolymerization of styrene or maleic anhydride, respectively, with p-glycidoxy-α-methyl styrene (I)

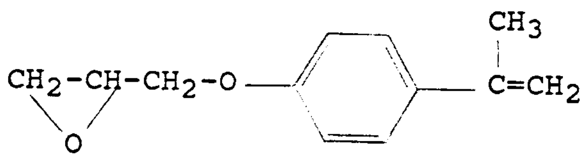

(I)

In US 3,327,019 there are described diethers which are the reaction product of p-glycidoxy styrenes and polyols. These compounds include aliphatic hydroxyl groups.

In Macromolecules, 16, 510-517 (1983), there is described the cationic polymerization of p-methoxy and p-(ethoxymethoxy)-α-methyl styrenes with boron trifluoride etherate as initiator in dichloromethane. These polymers are then subjected to ether cleavage reactions to yield linear polymers containing pendant phenolic groups.

It is known from kinetic studies of the cationic polymerization of p-methoxy styrene that this monomer has a very high rate of polymerization. See, e.g., Macromolecules, 9, 931-936 (1976); and Polymer, 16, 819-826 (1975).

United States Patent No. 3663625 (Neville) discloses bis[1-alkyl (and aryl)vinyl] para- phenylene oxide monomers of the formula (II)

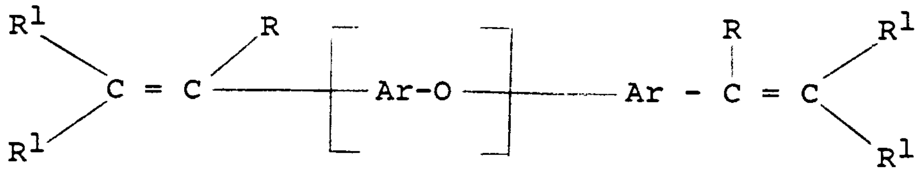

(II)

where Ar is a homocyclic aromatic structure selected from:

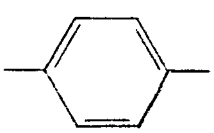

,

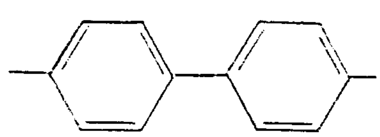

,

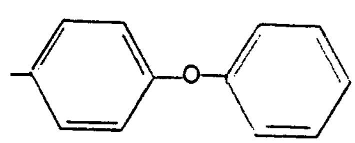

,

and

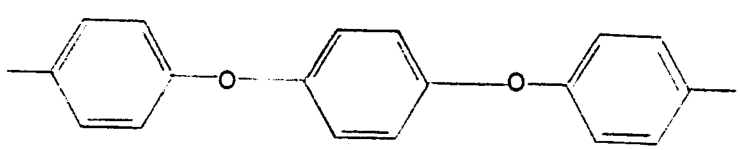

,

R is $C_{1-5}$ straight chain alkyl, biphenyl, naphthyl or $C_{1-5}$ fluoroalkyl;
$R^1$ is H or $C_{1-5}$ straight chain alkyl;
n is 1 to 6.

It is an essential feature of the monomers of US Patent 3663625 that they are substituted on the carbon atom adjacent to the group Ar (i.e. R cannot be hydrogen). It is stated that the monomers can be polymerized by means of free radical catalysts or Lewis acid catalysts. However, the object is to achieve linear polymers linked by indanyl structures. There is no suggestion of cationic addition polymerization.

Chemical Abstracts 63 9968 c-d (1965) discloses piperazine derivatives stated to be useful as analgesics and antipyretics and having the following structure (III)

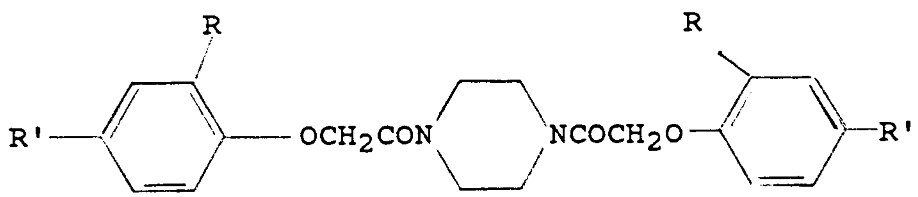
(III)

where R is methoxy or ethoxy and R' is methyl, ethyl, propyl, allyl or propenyl. There is no disclosure of any polymerization properties of the compounds and, in particular, no suggestion of cationic addition polymerization.

United States Patent No. 4,486,582 (Hefner) relates to the production of an ethylenically unsaturated monomer by reacting

(A) an aromatic material of the formula (IV)

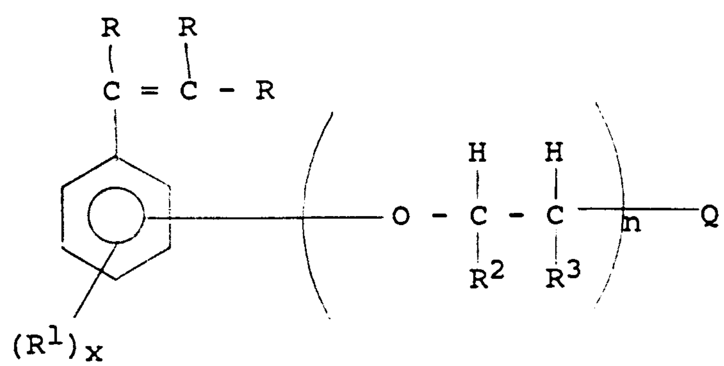
(IV)

wherein each R and $R^1$ group is independently selected from hydrogen or a saturated hydrocarbyl group having from 1 to 10 carbon atoms; each $R^2$ and $R^3$ is independently hydrogen or an alkyl group having from 1 to 4 carbon atoms; Q is a group having a hydrogen atom reactive with an -NCO and/or an -NCS group; n is 0 to 100 and x is 0 to 3;

(B) a material containing at least one oxyalkylene group and terminating in a group having at least one hydrogen atom which is reactive with -NCO and/or -NCS groups; and

(C) a material having an average of more than one -NCO and/or -NCS groups per molecule.

The monomers are thus urethanes or thiourethanes and they are stated to be reactive monomers which improve the properties, e.g. elongation, ductility and impact strength, of polyester and vinyl ester resins.

EP-A-0165769 (published 27th December, 1985) relates to cationically polymerizable styryloxy compounds of formula (V) as set out below in which G is a multi-valent organic or inorganic radical free of amino, aliphatic thiol, aliphatic hydroxyl, or other groups which interfere with cationic polymerization. There is no disclosure of the specific groups defined below for G.

The present invention is directed to a new class of cationic polymerizable monomers. In common with the monofunctional monomers discussed above, the inventive monomers contain styryloxy (p-vinylphenol ether) functionality.

According to one aspect, the present invention provides a method of forming a cross-linked solid polymer by addition polymerization of a polymerizable compound in the presence of a cationic initiator

characterized in that the polymerizable compound is selected from:

(A) a vinyl polymer having a plurality of groups within the backbone of the formula (V)

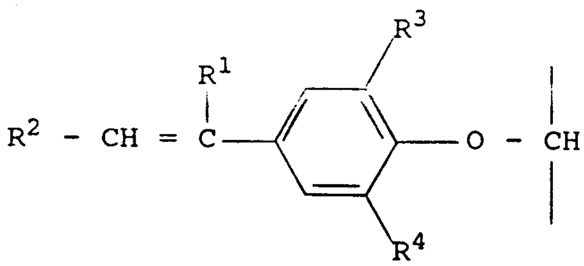

(V)

(B1) a compound of formula (VI)

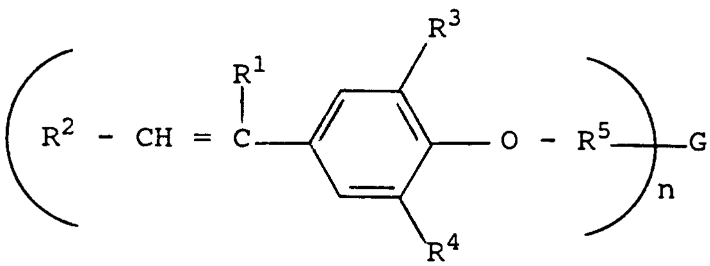

(VI)

where n is an integer of at least 2 and represents the number of groups within the brackets attached independently to G, and G is an n-valent radical free of amino, aliphatic hydroxyl, aliphatic thio or other groups which interfere with cationic polymerization and selected from:

(i) a radical comprising an n-valent polyphosphazene; and

(ii) a radical comprising an n-valent polyether;

(B2) a compound of formula (VII) or (VIII)

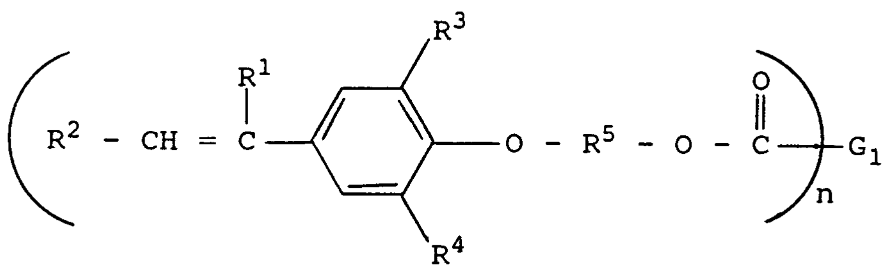

(VII)

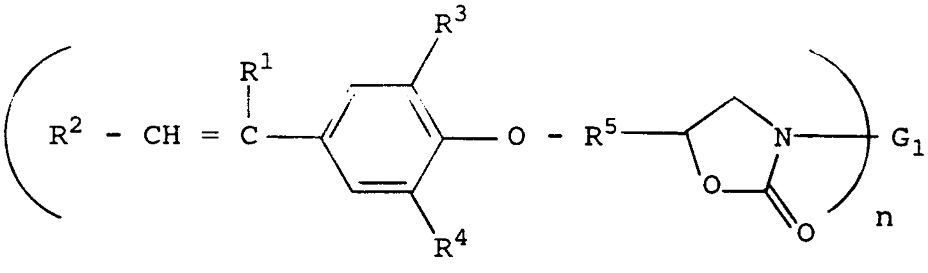

(VIII)

wherein n is an integer of at least 2 and represents the number of groups within the brackets attached

independently to $G_1$, and $G_1$ is an n-valent organic or inorganic radical free of amino, aliphatic hydroxyl, aliphatic thio or other groups which interfere with cationic polymerization; and

(C) a compound of formula (IX)

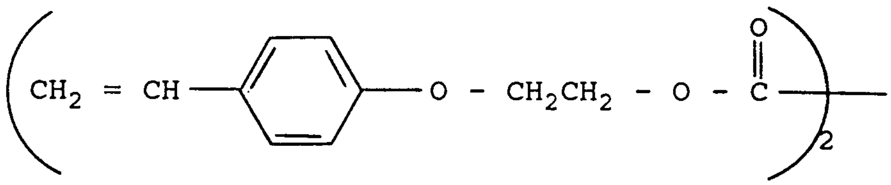

(IX)

wherein in the above formulae (V) to (VIII):

$R^1$ and $R^2$ are H, or one of $R^1$ and $R^2$ is H and the other is methyl;

$R^3$ and $R^4$ are H, $C_{1-4}$alkyl, or alkoxy if $R^2$ is not methyl; and

$R^5$ is a divalent hydrocarbon radical.

According to another aspect, the present invention provides a polyfunctional cationically polymerizable styryloxy compound selected from

(A) a vinyl polymer having a plurality of groups within the backbone of the formula (V)

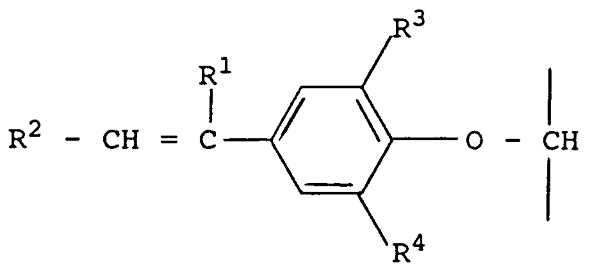

(V)

(B1) a compound of formula (VI)

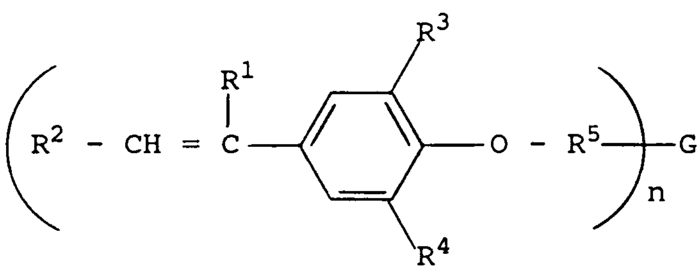

(VI)

where n is an integer of at least 2 and represents the number of groups within the brackets attached independently to G, and G is an n-valent radical free of amino, aliphatic hydroxyl, aliphatic thio or other groups which interfere with cationic polymerization and selected from:

(i) a radical comprising an n-valent polyphosphazene; and

(ii) a radical comprising an n-valent polyether;

(B2) a compound of formula (VII) or (VIII)

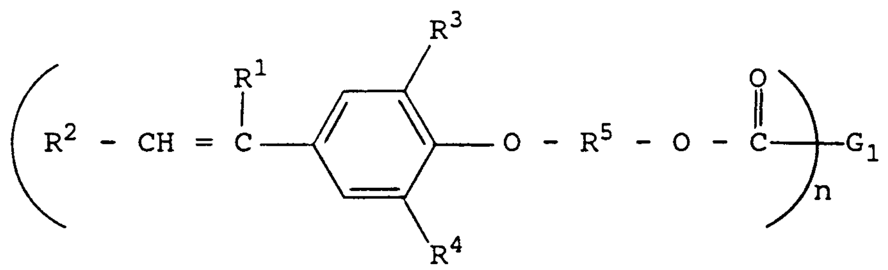

(VII)

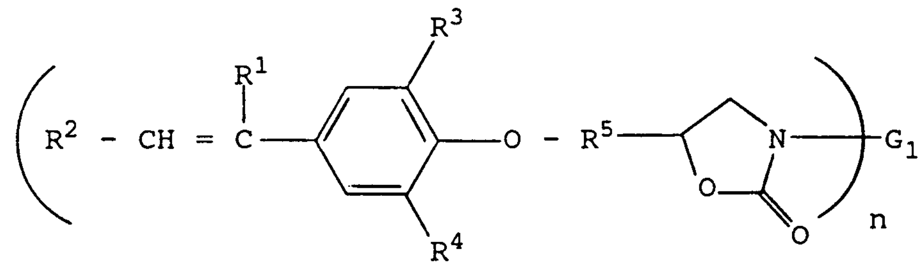

(VIII)

wherein n is an integer of at least 2 and represents the number of groups within the brackets attached independently to $G_1$, and $G_1$ is an n-valent organic or inorganic radical free of amino, aliphatic hydroxyl, aliphatic thio or other groups which interfere with cationic polymerization; and

(C) a compound of formula (IX)

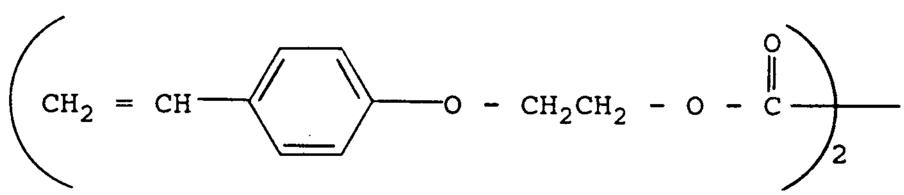

(IX)

wherein in the above formulae (V) to (VIII):

$R^1$ and $R^2$ are H, or one of $R^1$ and $R^2$ is H and the other is methyl;

$R^3$ and $R^4$ are H, $C_{1-4}$alkyl, or alkoxy if $R^2$ is not methyl; and

$R^5$ is a divalent hydrocarbon radical.

In addition to high reactivity to cationic polymerisations, the monomers defined above have been shown to develop an intense coloration when they are polymerized by UV irradiation in the presence of acid generating photoinitiators. Under some circumstances this coloration is sufficient to mask a substrate, providing a useful means of generating opacity in photocurable coatings. This coloration is also observed in chemically initiated cationic polymerization of these materials.

An additional feature of solid polyfunctional styryloxy resins of the invention is an ability of these resins to cure by UV irradiation without added photoinitiator. This cure is believed to involve a radical mechanism.

The monomers defined above may readily be prepared from p-vinyl phenols, p-propenyl phenols or p-isopropenyl phenols by a variety of methods such as etherification with an appropriate multi-functional etherifying agent, or reaction with multifunctional epoxies followed by reaction of the resulting aliphatic hydroxyl with a polyisocyanate or other suitable capping agent.

A suitable p-vinyl phenol is the commercially available vinyl guaiacol (2-methoxy-4-vinyl phenol). Synthetic methods for obtaining other suitable phenols include those reported in U.S. Patent 3,327,019, column 3, line 38 - column 4, line 2; Japanese Kokai Tokyo Koho 79:55,529 (dehydrogenation of ethyl phenol to give vinyl phenol); J. Kahovec, et al., J. Collect. Czech. Chem. Commun., 36, 1986 (1971) (various α-methylvinyl phenols); and Macromecules, l6 510-517 (1983)(p-hydroxy-α-methyl styrene by cleavage of

2,2-bis(p-hydroxyphenyl)-propane).

Yet another synthetic procedure involves the modification of the procedure of Macromolecules, 16, 510-517 (1983), in which p-(ethoxymethoxy)-α-methyl styrene is prepared from p-hydroxyacetophenone by etherifying the hydroxyl group and then using a Wittig reaction on the resulting p-(ethoxymethoxy) acetophenone. One modification required for the synthesis of the monomers defined above is the initial reaction of p-hydroxyacetophenone with an appropriate polyfunctional etherification reagent to form a molecule having multi-acetophenone functionalities. Subjecting such a molecule to a Wittig reaction as in the Macromolecules reference will yield a polyfunctional monomer of the invention.

An alternative modification of the Wittig reaction procedure is to use a monofunctional etherification reagent which is capable of entering into subsequent chain extension reactions with polyfunctional moieties.

In the formulas (V) and (VI) above, it is generally preferred that $R^3$ is H, methyl, or methoxy and $R^4$ is H. However, other lower alkyl or alkoxy (up to about $C_4$) may be included as substituents $R^3$ and/or $R^4$.

Examples of $R^5$ groups are methylene, ethylene or cycloaliphatic or aromatic hydrocarbons such as 1,4-dimethylenebenzene or unsaturated linear hydrocarbons such as propenylene or butenylene.

A limitation on G is that it must not interfere with cationic polymerization of the styryloxy groups. G must not include any strongly electron withdrawing group in conjugation with the styryloxy group oxygen atom as such groups will interfere with vinyl cationic polymerizations. Amines, aliphatic hydroxyls, and aliphatic thiols are known to prevent or slow vinyl cationic polymerizations. "Developments in Polymerization -1," R.N. Howard ed., Applied Science Publishers, 1979, pg. 80. Inclusion of these groups in G should therefore also be avoided.

Polymerization of the monomers defined above may be accomplished by conventional acid and Lewis acid cationic initiators such as methane sulfonic acid, toluene sulfonic acid and boron trifluoride etherate. UV cationic initiators may also be used. Such UV cationic photoinitiators include salts of a complex halogenide having the formula:

$$[A]^{+}_{d}[MX_{e}]^{-(e-f)}$$

where A is a cation selected from the group consisting of iodonium, sulfonium, pyrylium, thiopyrylium and diazonium cations, M is a metalloid, and X is a halogen radical, d equals e minus f, f equals the valance of M and is an integer equal to from 2 to 7 inclusive, e is greater than f and is an integer having a value up to 8. Examples include di-p-tolyl iodonium hexafluorophosphate, diphenyl iodonium hexafluorophosphate, diphenyl iodonium hexafluoroarsenate and UVE 1014 (trademark of General Electric), a commercially available sulfonium salt of a complex halogenide.

Certain monomers, usually solids, will also undergo UV initiated polymerization in the solid state without initiator, yielding an essentially uncolored product. A radical mechanism is believed to be involved.

The production of colored reaction mixtures by cationic initiators has been reported before for styryloxy monomers. Permanent coloration in the cured products of the invention is believed to result from particular termination reactions involving stable carbocations. The development of color can thus be controlled by selecting polymerization conditions designed to select for or against termination by stable carbocations. The development of permanent color as a result of polymerization termination reactions is especially advantageous at certain UV cured opaque coating applications where the use of pigments or dyes in the composition blocks UV, resulting in only surface cure of the coating. Since the inventive resins develop their intense coloration only after initiation of polymerization, initiation by UV is not interfered with. The invention may be illustrated by reference to the following nonlimiting examples:

EXAMPLE 1

Vinyl polymer with Styryloxy Groups:

To a mixture of 1.5 equivalents $K_2CO_3$ dispersed in acetone is added 1.1 equivalent p-hydroxybenzaldehyde and sufficient polyvinyl chloride or vinyl chloride copolymer to provide approximately 1 equivalent of allylic chloride groups (i.e. - CH = CH - CHCl -). The mixture is refluxed for about 4 hours after which the inorganic salts are filtered and the polymer precipitated with hexane or petroleum ether and dried. The resulting polymer has multiple groups within its backbone of the structure:

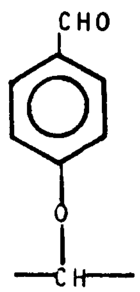

The aldehyde equivalence of the polymer may be determined spectrophotometrically.

The aldehyde containing polymer is then dissolved in tetrahydrofuran (THF) and a Wittig reagent soln, prepared by mixing equimolar amounts of methyltriphenylphosphonium bromide and sodium amide and filtering out the inorganic salts, is added dropwise until an approximately 10% excess of the Wittig reagent has been added.

The mixture is then refluxed for about 4 hours while $N_2$ is bubbled into the mixture. After the solution has been allowed to return to room temperature, methanol is added to precipitate the product. The product is collected dried and identified as a vinyl chloride copolymer having plural groups within its backbone of the formula:

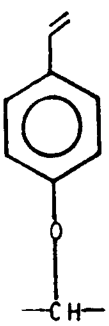

## EXAMPLE 2

Two equivalents of the monoethoxylate of 4-hydroxybenzaldehyde is reacted with 1 mole oxalylchloride followed by Wittig reaction on the aldehyde functionalities to give

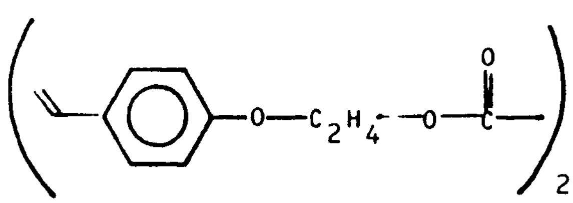

Other di or polyesters may be prepared by analogous procedures, using, for instance, sebacyl chloride, phthalyl chloride, the tetrachloride of benzophenone tetracarboxylic acid, etc., or the corresponding acid anhydrides.

## EXAMPLE 3

The monoethoxylate of 4-hydroxybenzaldehyde is esterified with 2-mercapto acetic acid followed by Wittig reaction to give a thiol functional styryloxy ester of the formula:

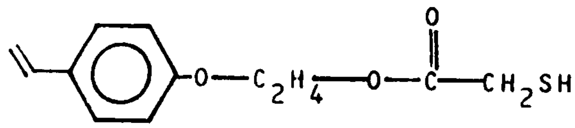

The thiol may then be reacted with a plural functional thiol reactive compound such as a di- or poly-isocyanate to give a multifunctional styryloxy resin within the invention.

EXAMPLE 4

4-hydroxy benzaldehyde is reacted with epichlorohydrin in the presence of potassium carbonate as etherification catalyst to give the glycidyl ether of 4-hydroxybenzaldehyde. This ether is polymerized cationically to give a polyether polymer, substantially free of hydroxyls, having repeat units of the formula:

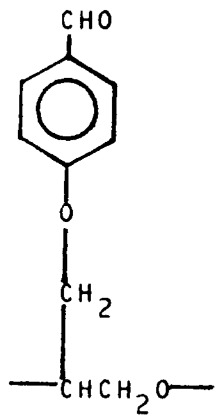

Wittig reaction on this polymer gives the corresponding cationically cureable styryloxy functional polyether.

EXAMPLE 5

The glycidyl ether of 4-hydroxybenzaldehyde is reacted with toluene diisocyanate in the presence of $ZnBr_2/(C_4H_9)_4PO$ catalyst at a ratio of 2 moles aldehyde to each mole diisocyanate under standard oxazolidone synthesis conditions to give a cationically polymerizable resin of the formula:

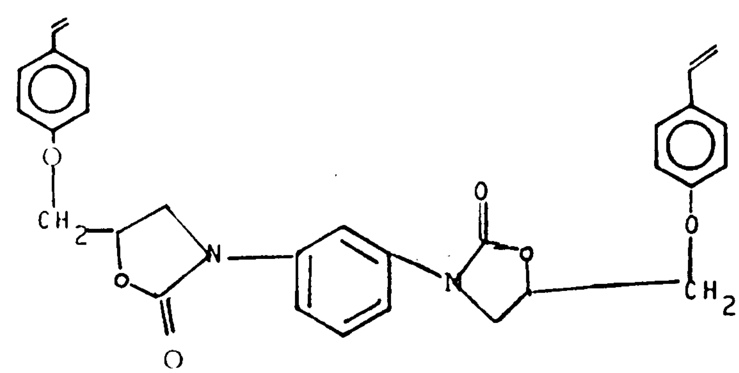

Similar resins can be prepared from other diisocyanate or polyisocyanate functional materials.

EXAMPLE 6

The monoethoxylate of 4-hydroxybenzaldehyde is reacted with a phosphazene polymer or cyclic oligomer having plural repeat units of the formula:

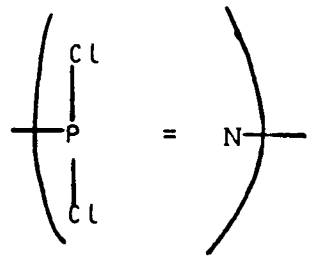

at a ratio of 2 equivalents of aldehyde per repeat unit of the formula above. The reaction is run in THF in the presence of a 10% molar excess (based on aldehyde content) of triethylamine in a sealed tube at 100 ° C for 24 hours.

The ammonium chloride produced by the reaction is filtered from the cooled solution and the polymer isolated by evaporation of the solvent. The aldehyde functionality is then subjected to Wittig reaction to give a phosphazene polymer with plural units of the formula:

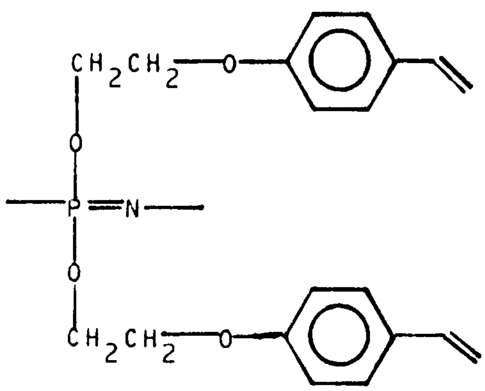

Other products of the invention having phosphazene backbones may also be prepared by methods which will be apparent to those skilled in the art, including copolymers in which some of the P-Cl bonds are substituted with another organic group before reaction with the ethoxylated benzaldehyde. Other benzaldehydes may also be used, for instance, the monoethylenehydroxy group may be replaced by other alkylene hydroxy groups, the carbon atoms of which may be optionally interrupted with one or more oxygen atoms.

Aldehyde compounds of this type may be represented by the formula:

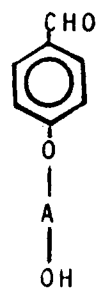

where A is alkylene, alkyleneoxy alkylene or (poly alkyleneoxy) alkylene.

**Claims**

1. A method of forming a cross-linked solid polymer by addition polymerization of a polymerizable compound in the presence of a cationic initiator characterized in that the polymerizable compound is selected from:

(A) a vinyl polymer having a plurality of groups within the backbone of the formula (V)

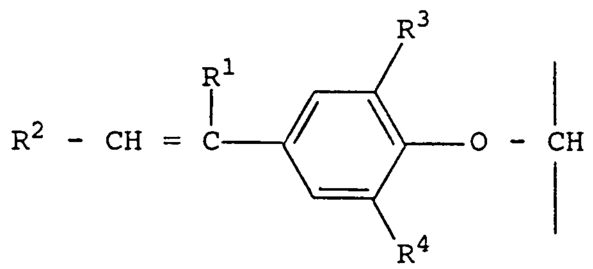

(V)

(B1) a compound of formula (VI)

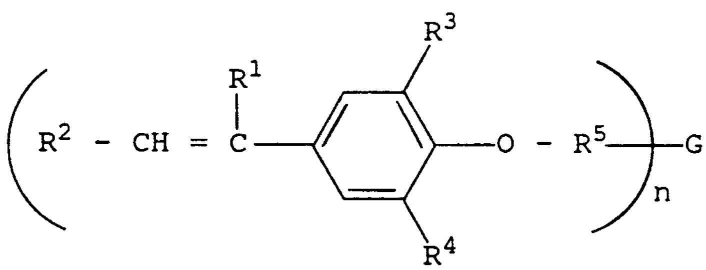

(VI)

where n is an integer of at least 2 and represents the number of groups within the brackets attached independently to G, and G is an n-valent radical free of amino, aliphatic hydroxyl, aliphatic thio or other groups which interfere with cationic polymerization and selected from:

(i) a radical comprising an n-valent polyphosphazene; and

(ii) a radical comprising an n-valent polyether;

(B2) a compound of formula (VII) or (VIII)

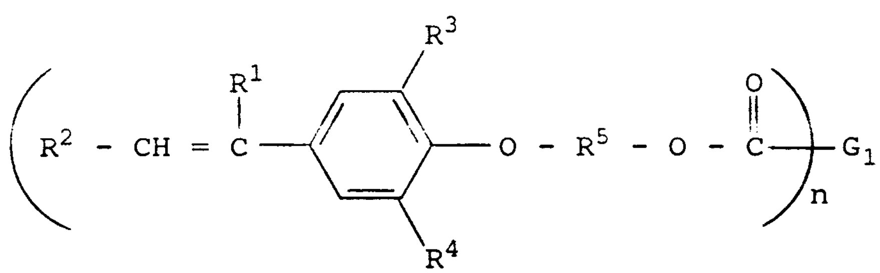

(VII)

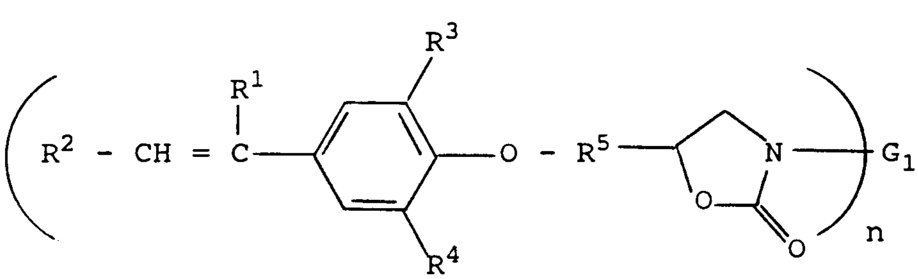

(VIII)

wherein n is an integer of at least 2 and represents the number of groups within the brackets attached independently to $G_1$, and $G_1$ is an n-valent organic or inorganic radical free of amino, aliphatic hydroxyl, aliphatic thio or other groups which interfere with cationic polymerization; and

(C) a compound of formula (IX)

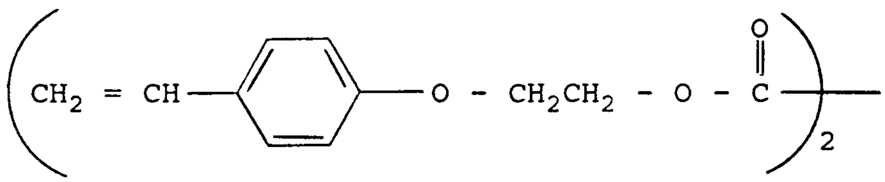

(IX)

wherein in the above formulae (V) to (VIII):

$R^1$ and $R^2$ are H, or one of $R^1$ and $R^2$ is H and the other is methyl;

$R^3$ and $R^4$ are H, $C_{1-4}$alkyl, or alkoxy if $R^2$ is not methyl; and

$R^5$ is a divalent hydrocarbon radical.

**2.** A method as claimed in Claim 1 characterised in that the initiator is a photoinitiator and the polymerizable compound is exposed to ultra violet light.

**3.** A polyfunctional cationically polymerizable styryloxy compound selected from

(A) a vinyl polymer having a plurality of groups within the backbone of the formula (V)

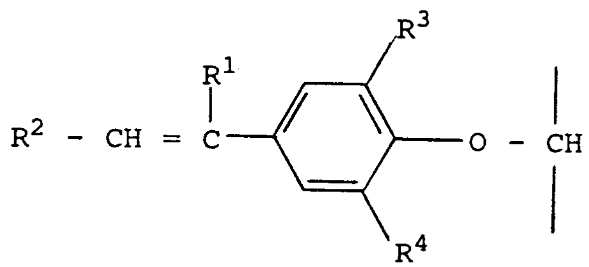

(V)

(B1) a compound of formula (VI)

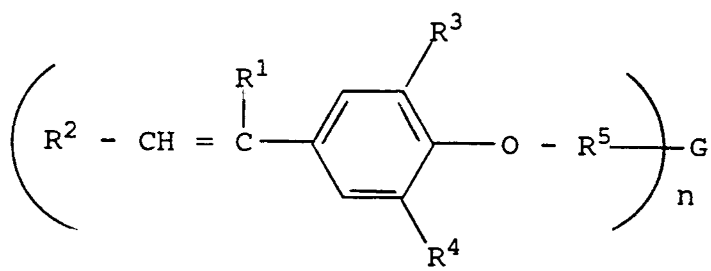

(VI)

where n is an integer of at least 2 and represents the number of groups within the brackets attached independently to G, and G is an n-valent radical free of amino, aliphatic hydroxyl, aliphatic thio or other groups which interfere with cationic polymerization and selected from:

(i) a radical comprising an n-valent polyphosphazene; and

(ii) a radical comprising an n-valent polyether;

(B2) a compound of formula (VII) or (VIII)

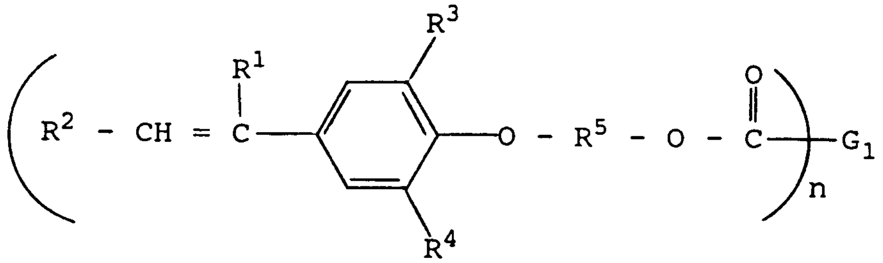

(VII)

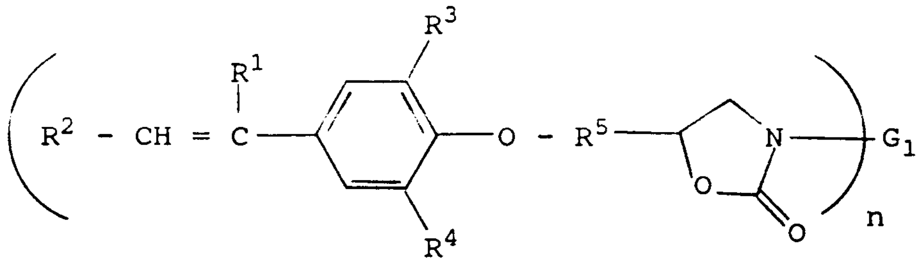

(VIII)

wherein n is an integer of at least 2 and represents the number of groups within the brackets attached independently to $G_1$, and $G_1$ is an n-valent organic or inorganic radical free of amino, aliphatic hydroxyl, aliphatic thio or other groups which interfere with cationic polymerization; and

(C) a compound of formula (IX)

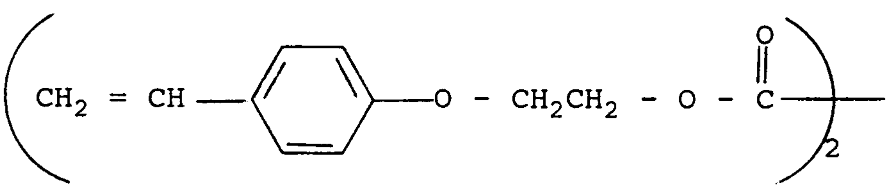

(IX)

wherein in the above formulae (V) to (VIII):

$R^1$ and $R^2$ are H, or one of $R^1$ and $R^2$ is H and the other is methyl;

$R^3$ and $R^4$ are H, $C_{1-4}$alkyl, or alkoxy if $R^2$ is not methyl; and

$R^5$ is a divalent hydrocarbon radical.

**4.** A compound as claimed in Claim 3 characterised in that $R^3$ is H, methyl or methoxy, and $R^4$ is H.

**5.** A compound as claimed in Claim 3 or 4, characterised in that $R^5$ is a divalent hydrocarbon radical selected from methylene, ethylene, cycloaliphatic hydrocarbon, aromatic hydrocarbon and unsatuated linear hydrocarbon.

**6.** A compound as claimed in Claim 5, characterised in that $R^5$ is 1,4-dimethylenebenzene, propenylene or butenylene.

**Patentansprüche**

**1.** Verfahren zur Bildung eines vernetzten festen Polymeren durch Additionspolymerisation einer polymerisierbaren Verbindung in Gegenwart eines kationischen Starters, dadurch gekennzeichnet, daß die polymerisierbare Verbindung ausgewählt wird aus:

(A) einem Vinyl-Polymeren, das in der Hauptkette eine Vielzahl von Gruppen der Formel (V) aufweist

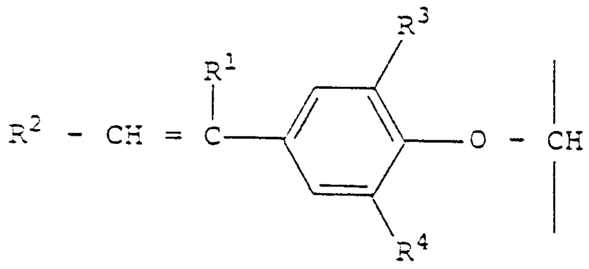

(V)

(B1) einer Verbindung der Formel (VI)

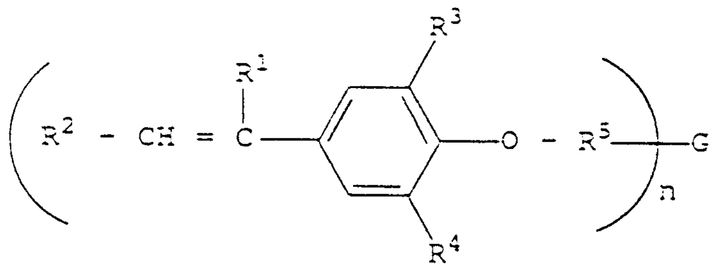

(VI)

worin n eine ganze Zahl von wenigstens 2 bedeutet und die Anzahl der Gruppen in Klammern darstellt, die unabhängig an G angeknüpft sind, und G ein n-wertiges Radikal, frei von Amino, aliphatischem Hydroxy, aliphatischem Thio oder weiteren Gruppen, die die kationische Polymerisation stören, darstellt und ausgewählt wird aus:

(i) einem Radikal, umfassend ein n-wertiges Polyphosphazen; und

(ii) einem Radikal, umfassend einen n-wertigen Polyether;

(B2) einer Verbindung der Formel (VII) oder (VIII)

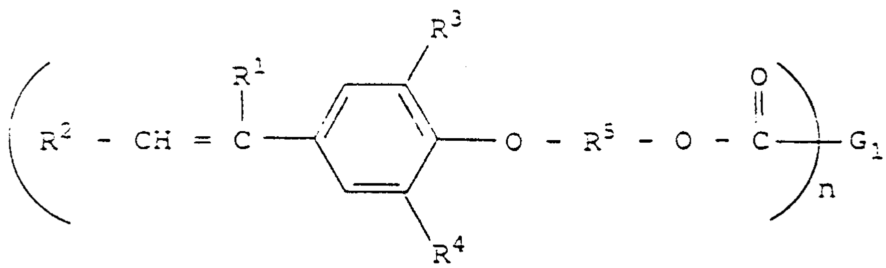

(VII)

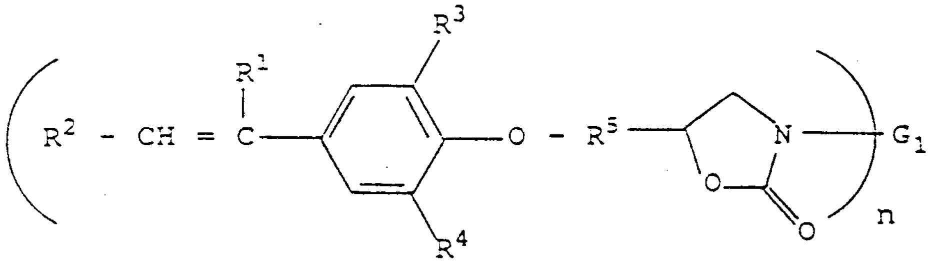

(VIII)

worin n eine ganze Zahl von wenigstens 2 bedeutet und die Anzahl der Gruppen in Klammern darstellt, die unabhängig an $G_1$ angeknüpft sind, und $G_1$ ein n-wertiges organisches oder anorganisches Radikal, frei von Amino, aliphatischem Hydroxy, aliphatischem Thio oder weiteren Gruppen, die die kationische Polymerisation stören, darstellt; und

(C) einer Verbindung der Formel (IX)

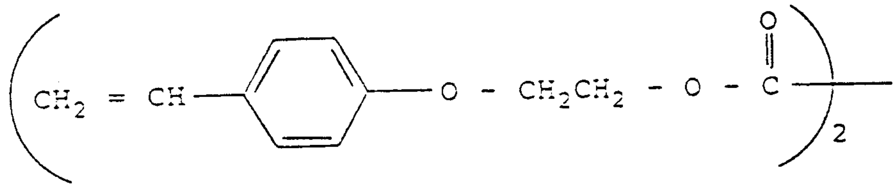

(IX)

worin in den obigen Formeln (V) bis (VIII):
$R^1$ und $R^2$ H bedeuten oder eines von $R^1$ und $R^2$ H und das andere Methyl bedeutet;
$R^3$ und $R^4$ H, $C_{1-4}$-Alkyl oder Alkoxy bedeuten, wenn $R^2$ nicht Methyl bedeutet; und
$R^5$ für ein zweiwertiges Kohlenwasserstoff-Radikal steht.

**2.** Verfahren nach Anspruch 1, dadurch gekennzeichnet, daß der Starter ein Photostarter ist und die polymerisierbare Verbindung gegenüber ultraviolettem Licht ausgesetzt wird.

**3.** Polyfunktionelle kationisch polymerisierbare Styryloxy-Verbindung, ausgewählt aus
(A) einem Vinyl-Polymeren, das in der Hauptkette eine Vielzahl von Gruppen der Formel (V) aufweist

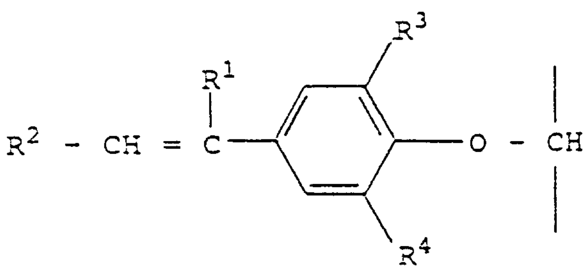

(V)

(B1) einer Verbindung der Formel (VI)

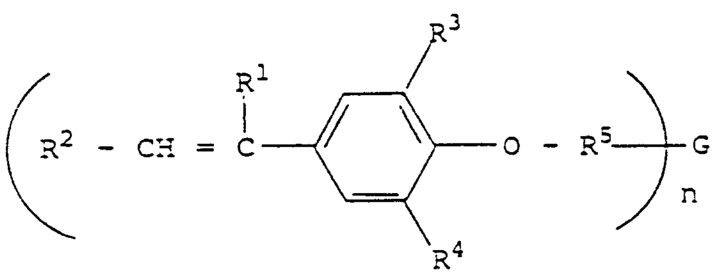

(VI)

worin n eine ganze Zahl von wenigstens 2 bedeutet und die Anzahl der Gruppen in Klammern darstellt, die unabhängig an G angeknüpft sind, und G ein n-wertiges Radikal, frei von Amino, aliphatischem Hydroxy, aliphatischem Thio oder weiteren Gruppen, die die kationische Polymerisation stören, darstellt und ausgewählt wird aus:
(i) einem Radikal, umfassend ein n-wertiges Polyphosphazen; und
(ii) einem Radikal, umfassend einen n-wertigen Polyether;

(B2) einer Verbindung der Formel (VII) oder (VIII)

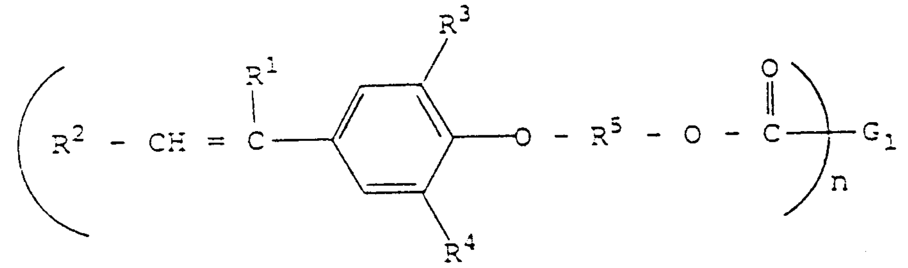

(VII)

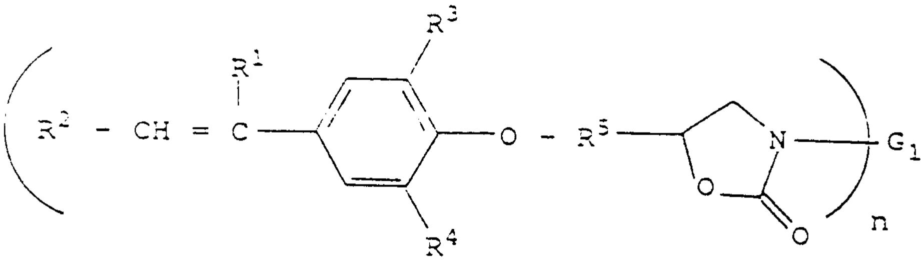

(VIII)

worin n eine ganze Zahl von wenigstens 2 bedeutet und die Anzahl der Gruppen in Klammern darstellt, die unabhängig an $G_1$ angeknüpft sind, und $G_1$ ein n-wertiges organisches oder anorganisches Radikal, frei von Amino, aliphatischem Hydroxy, aliphatischem Thio oder weiteren Gruppen, die die kationische Polymerisation stören, darstellt; und

(C) einer Verbindung der Formel (IX)

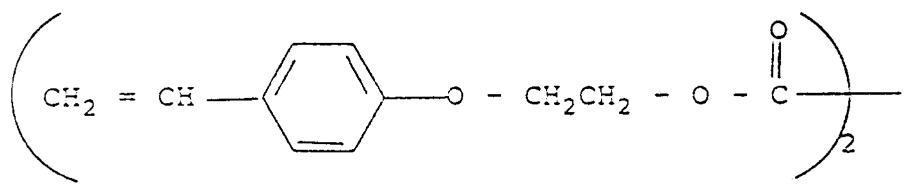

(IX)

worin in den obigen Formeln (V) bis (VIII):

$R^1$ und $R^2$ für H stehen oder eines von $R^1$ und $R^2$ H und das andere Methyl bedeutet;

$R^3$ und $R^4$ für H, $C_{1-4}$-Alkyl oder Alkoxy stehen, wenn $R^2$ nicht Methyl bedeutet; und

$R^5$ für ein zweiwertiges Kohlenwasserstoff-Radikal steht.

**4.** Verbindung nach Anspruch 3, dadurch gekennzeichnet, daß $R^3$ für H, Methyl oder Methoxy steht und $R^4$ H bedeutet.

**5.** Verbindung nach Anspruch 3 oder 4, dadurch gekennzeichnet, daß $R^5$ ein zweiwertiges Kohlenwasserstoff-Radikal darstellt, ausgewählt aus Methylen, Ethylen, einem cycloaliphatischen Kohlenwasserstoff, einem aromatischen Kohlenwasserstoff und einem ungesättigten linearen Kohlenwasserstoff.

**6.** Verbindung nach Anspruch 5, dadurch gekennzeichnet, daß $R^5$ für 1,4-Dimethylenbenzol, Propenylen oder Butenylen steht.

**Revendications**

**1.** Un procédé de formation d'un polymère solide réticulé par polymérisation additive d'un composé polymérisable en présence d'un agent initiateur cationique, caractérisé en ce que le composé polymérisable est choisi parmi :

(A) un polymère vinylique dont le squelette comporte plusieurs groupes de formule (V)

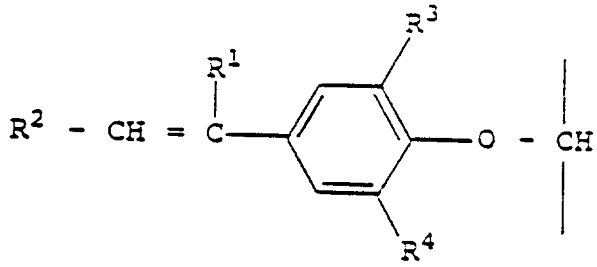

(V)

(B1) un composé de formule (VI)

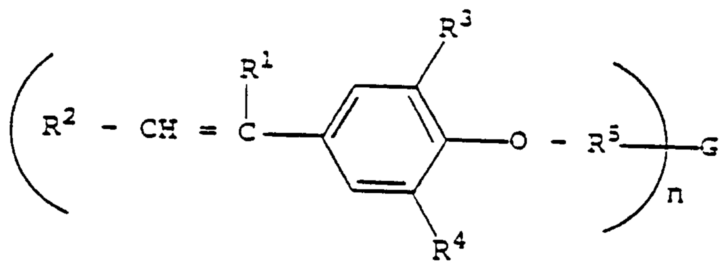

(VI)

où n est un entier au moins égal à 2 et représente le nombre de groupes entre les parenthèses attachés indépendamment à G, et G est un radical n-valent dépourvu de groupes amino, hydroxy aliphatiques, thio aliphatiques ou autres groupes faisant obstacle à la polymérisation cationique et est choisi parmi :

(i) un radical comprenant un polyphosphazène n-valent; et

(ii) un radical comprenant un polyéther n-valent;

(B2) un composé de formule (VII) ou (VIII)

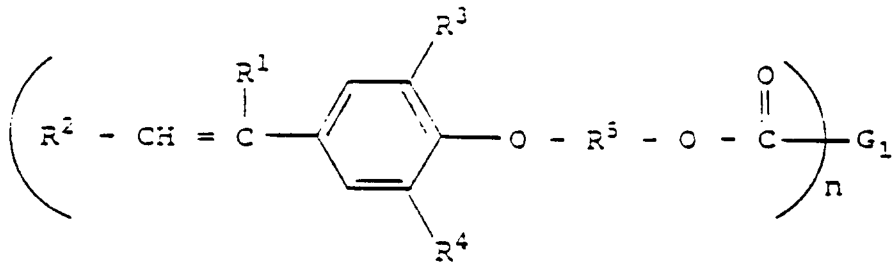

(VII)

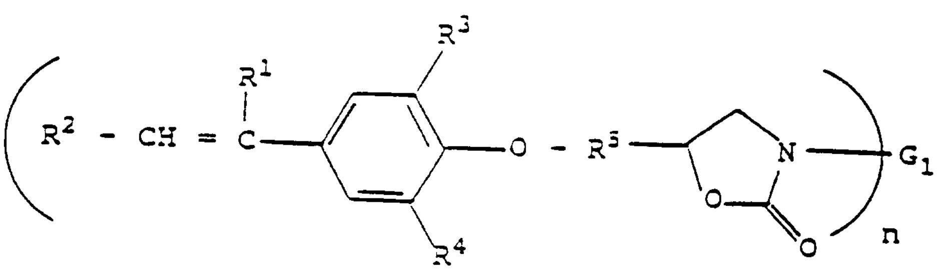

(VIII)

où n est un entier au moins égal à 2 et représente le nombre de groupes entre les parenthèses attachés indépendamment à $G_1$, et $G_1$ est un radical organique ou minéral n-valent dépourvu de groupes amino, hydroxy aliphatiques, thio aliphatiques ou d'autres groupes faisant obstacle à la polymérisation cationique; et

(C) un composé de formule (IX)

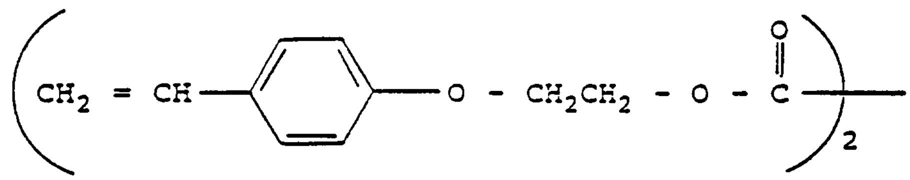

(IX)

où, dans les formules (V) à (VII) ci-dessus :

$R^1$ et $R^2$ sont des atomes d'hydrogène, ou l'un des groupes $R^1$ et $R^2$ est un atome d'hydrogène et l'autre est un groupe méthyle;

$R^3$ et $R^4$ sont des atomes d'hydrogène, des groupes alkyles en $C_1$ à $C_4$ ou des groupes alcoxy si $R^2$ n'est pas un groupe méthyle; et

$R^5$ est un radical hydrocarboné divalent.

**2.** Un procédé selon la revendication 1 caractérisé en ce que l'agent initiateur est un agent photoinitiateur et en ce que le composé polymérisable est exposé à de la lumière ultraviolette.

**3.** Un composé styryloxy polyfonctionnel polymérisable cationiquement choisi parmi

(A) un polymère vinylique dont le squelette comporte plusieurs groupes de formule (V)

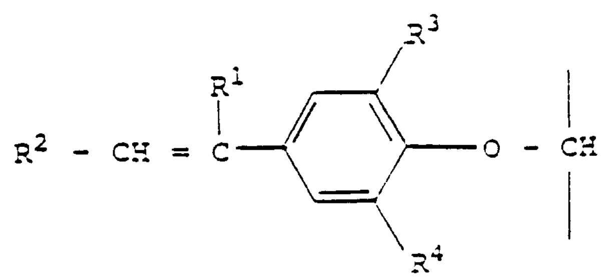

(V)

(B1) un composé de formule (VI)

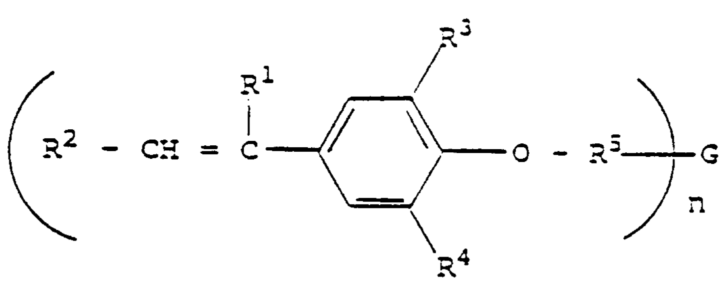

(VI)

où n est un entier au moins égal à 2 et représente le nombre de groupes entre les parenthèses attachés indépendamment à G, et G est un radical n-valent dépourvu de groupes amino, hydroxy aliphatiques, thio aliphatiques ou autres groupes faisant obstacle à la polymérisation cationique et est choisi parmi :

(i) un radical comprenant un pholyphosphazène n-valent; et

(ii) un radical comprenant un polyéther n-valent;

(B2) un composé de formule (VII) ou (VIII)

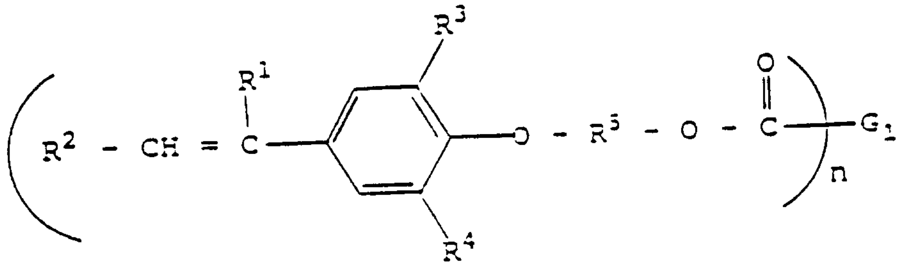

(VII)

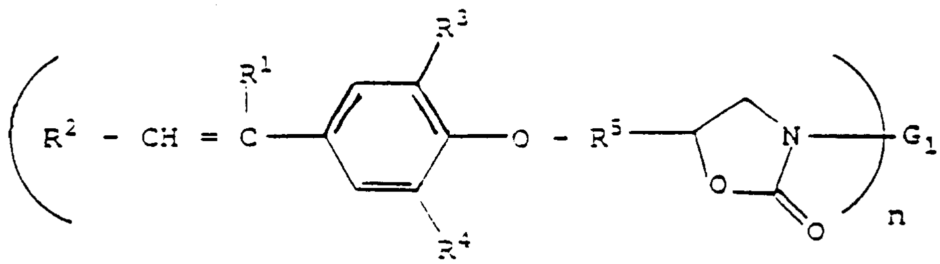

(VIII)

où n est un entier au moins égal à 2 et représente le nombre de groupes entre les parenthèses attachés indépendamment à $G_1$, et $G_1$ est un radical organique ou minéral n-valent dépourvu de groupes amino, hydroxy aliphatiques, thio aliphatiques ou d'autres groupes faisant obstacle à la polymérisation cationique; et

(C) un composé de formule (IX)

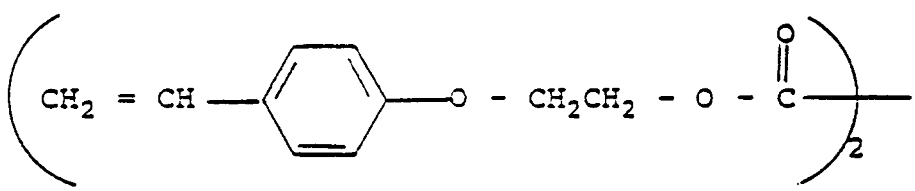

(IX)

où, dans les formules (V) à (VIII) ci-dessus :

$R^1$ et $R^2$ sont des atomes d'hydrogène, ou l'un des groupes $R^1$ et $R^2$ est un atome d'hydrogène et l'autre est un groupe méthyle;

$R^3$ et $R^4$ sont des atomes d'hydrogène, des groupes alkyles en $C_1$ à $C_4$, ou des groupes alcoxy si $R^2$ n'est pas un groupe méthyle; et

$R^5$ est un radical hydrocarboné divalent.

**4.** Un composé selon la revendication 3 caractérisé en ce que $R^3$ est un atome d'hydrogène, un groupe méthyle ou un groupe méthoxy, et $R^4$ est un atome d'hydrogène.

**5.** Un composé selon la revendication 3 ou 4, caractérisé en ce que $R^5$ est un radical hydrocarboné divalent choisi parmi le groupe méthylène, le groupe éthylène, les groupes hydrocarbonés cycloaliphatiques, les groupes hydrocarbonés aromatiques et les groupes hydrocarbonés linéaires insaturés.

**6.** Un composé selon la revendication 5, caractérisé en ce que $R^5$ est un groupe 1,4-diméthylènebenzène, propénylène ou buténylène.